(19) Europäisches Patentamt
European Patent Office
Office européen des brevets

(11) **EP 3 309 575 A1**

(12) **EUROPEAN PATENT APPLICATION**

(43) Date of publication:
**18.04.2018 Bulletin 2018/16**

(51) Int Cl.:
*G01R 33/561* (2006.01)    *G01R 33/56* (2006.01)
*G01R 33/567* (2006.01)

(21) Application number: **17195936.4**

(22) Date of filing: **11.10.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **12.10.2016 KR 20160132144**

(71) Applicant: **Samsung Electronics Co., Ltd.
Gyeonggi-do 16677 (KR)**

(72) Inventor: **CHOI, Sang-cheon
103-403 Gyeonggi-do (KR)**

(74) Representative: **Jacobs, Bart et al
Arnold & Siedsma
Bezuidenhoutseweg 57
2594 AC The Hague (NL)**

(54) **INTERLEAVED MULTI-SLICE DOUBLE INVERSION-RECOVERY MAGNETIC RESONANCE IMAGING WITH MULTIPLE CONTRASTS**

(57)    A magnetic resonance imaging (MRI) apparatus, including a processor and a memory connected to the processor, wherein the processor is configured to control a radio frequency (RF) coil to apply a first pulse sequence to a first slice from among a plurality of slices of an object during a first RR interval of a heart, and acquire a first magnetic resonance (MR) signal corresponding to the first pulse sequence from the RF coil, control the RF coil to apply a second pulse sequence to a second slice from among the plurality of slices during a second RR interval of the heart, and acquire a second MR signal corresponding to the second pulse sequence from the RF coil, reconstruct a first MR image from the first MR signal, and reconstruct a second MR image from the second MR signal. The first and the second pulse sequence may include different double inversion recovery pulse sequences so that a blood signal is supressed and a different image contrast is created for the first and the second magnetic resonance image.

FIG. 8

EP 3 309 575 A1

**Description**

**1. Field**

Exemplary embodiments consistent with the present disclosure relate to a magnetic resonance imaging (MRI) apparatus and a method of operating the MRI apparatus.

**2. Description of Related Art**

A magnetic resonance imaging (MRI) apparatus uses a magnetic field to capture an image of an object. An MRI apparatus may be used for accurate disease diagnosis because stereoscopic images of bones, lumbar discs, joints, nerves, ligaments, the heart, etc. can be obtained at desired angles.

When imaging, for example, a heart of an object using the MRI apparatus, a scan may be is long, and due to image distortion occurring due to a movement of the heart, it may be difficult to recognize a lesion. Thus, in order for the MRI apparatus to easily recognize a lesion of the heart, it is required to simultaneously generate an image with anatomy information of the heart and an image for recognizing the lesion of the heart.

SUMMARY

Provided are a magnetic resonance imaging (MRI) apparatus that reconstructs a plurality of magnetic resonance (MR) images having different contrasts during a scan period, and a method of operating the MRI apparatus.

Additional aspects will be set forth in part in the description which follows and, in part, will be apparent from the description, or may be learned by practice of the presented exemplary embodiments.

According to an aspect of an exemplary embodiments, a magnetic resonance imaging (MRI) apparatus includes a processor and a memory connected to the processor, wherein the processor is configured to control a radio frequency (RF) coil to apply a first pulse sequence to a first slice from among a plurality of slices of an object during a first RR interval of a heart, and acquire a first magnetic resonance (MR) signal corresponding to the first pulse sequence from the RF coil, control the RF coil to apply a second pulse sequence to a second slice from among the plurality of slices during a second RR interval of the heart, and acquire a second MR signal corresponding to the second pulse sequence from the RF coil, reconstruct a first MR image from the first MR signal, and reconstruct a second MR image from the second MR signal.

The first pulse sequence may use a long echo time (TE), and the second pulse sequence may use a short TE.

The first pulse sequence may include a first inversion pulse sequence for suppressing a blood signal in the first slice, and a first excitation pulse sequence for acquiring the first MR signal, and the second pulse sequence may include a second inversion pulse sequence for suppressing the blood signal in the first slice, and a second excitation pulse sequence for acquiring the second MR signal.

In the first inversion pulse sequence, the processor may be further configured to control the RF coil to apply a first inversion RF pulse to the object for inverting a magnetization of the object, and to apply a second inversion RF pulse to the first slice for recovering the magnetization inverted by the first inversion RF pulse.

Iin the second inversion pulse sequence, the processor may be further configured to control the RF coil to apply a first inversion RF pulse to the object to invert magnetization of the object, and to apply a second inversion RF pulse to the first slice and the second slice to recover the magnetization inverted by the first inversion RF pulse.

The first MR image may be a T2-weighted image and the second MR image may be a T1-weighted image.

In the first excitation pulse sequence, the processor may be further configured to control the RF coil to apply to the first slice a third inversion RF pulse for suppressing a fat signal, and to apply to the first slice at least one RF excitation pulse for acquiring the first MR signal.

In the first inversion pulse sequence, when the second pulse sequence uses a long repetition time (TR), the processor may be further configured control the RF coil to apply to the object a first inversion RF pulse for inverting magnetization of the object, and to apply to the first slice and the second slice a second inversion RF pulse for recovering the magnetization inverted by the first inversion RF pulse.

The first MR image may be a T2-weighted image and the second MR image may be a proton density (PD)-weighted image.

A position of the second slice may be discontinuous with respect to a position of the first slice.

According to another aspect of an exemplary embodiment, a method of operating a magnetic resonance imaging (MRI) apparatus includes applying, using a radio-frequency (RF) coil, a first pulse sequence to a first slice from among a plurality of slices of an object during a first RR interval of a heart, acquiring, using the RF coil, a first magnetic resonance (MR) signal corresponding to the first pulse sequence, applying, using the RF coil, a second pulse sequence to a second slice from among the plurality of slices during a second RR interval of the heart, acquiring, using the RF coil, a second MR signal corresponding to the first pulse sequence, reconstructing a first MR image from the first MR signal, and reconstructing a second MR image from the second MR signal.

The first pulse sequence may use a long echo time (TE), and the second pulse sequence may use a short TE.

The first pulse sequence may include a first inversion pulse sequence for suppressing a blood signal in the first slice, and a first excitation pulse sequence for acquiring the first MR signal, and the second pulse sequence may include a second inversion pulse sequence for suppressing the blood signal in the first slice, and a second excitation pulse sequence for acquiring the second MR signal.

The acquiring of the first MR signal may include, in the first inversion pulse sequence, applying to the object a first inversion radio frequency (RF) pulse for inverting magnetization of the object, and applying to the first slice a second inversion RF pulse for recovering the magnetization inverted by the first inversion RF pulse, and acquiring the first MR signal corresponding to the first slice, based on the first excitation pulse sequence.

The acquiring of the first MR signal corresponding to the first slice, based on the first excitation pulse sequence, may include applying, to the first slice, a third inversion RF pulse for suppressing a fat signal, and applying at least one excitation RF pulse to the first slice.

The acquiring of the second MR signal may include, in the second inversion pulse sequence, applying a first inversion RF pulse to the object to invert magnetization of the object, and applying a second inversion RF pulse to the first slice and the second slice to recover the magnetization inverted by the first inversion RF pulse, and applying at least one RF excitation pulse to the second slice, based on the second excitation pulse sequence.

The first MR image may be a T2-weighted image and the second MR image may be a T1-weighted image.

When the second pulse sequence uses a long repetition time (TR), the acquiring of the first MR signal may include, in the first inversion pulse sequence, applying to the object a first inversion RF pulse for inverting magnetization of the object, and applying, to the first slice and the second slice, a second inversion RF pulse for recovering the magnetization inverted by the first inversion RF pulse, and acquiring the first MR signal corresponding to the first slice, based on the first excitation pulse sequence.

The first MR image may be a T2-weighted image and the second MR image may be a proton density (PD)-weighted image.

According to another aspect of an exemplary embodiment, a computer-readable recording medium may have recorded thereon a program which, when executed, may cause a processor to perform the method of claim 11.

According to another aspect of an exemplary embodiment, a magnetic resonance imaging (MRI) device for obtaining magnetic resonance (MR) images of an object including a heart includes a radio-frequency (RF) coil configured to apply a first pulse sequence to a first slice from among a plurality of slices of the object during a first RR interval of the heart, acquire a first MR signal corresponding to the first pulse sequence, apply a second pulse sequence to a second slice from among the plurality of slices during a second RR interval of the heart, and acquire a second MR signal corresponding to the second pulse sequence, and a processor configured to reconstruct a first MR image from the first MR signal, and reconstruct a second MR image from the second MR signal, wherein the second slice is discontinuous with respect to the first slice, so that atomic nuclei in the first slice which are excited by the first pulse sequence are allowed to relax during the second RR interval.

BRIEF DESCRIPTION OF THE DRAWINGS

These and/or other aspects will become apparent and more readily appreciated from the following description of exemplary embodiments, taken in conjunction with the accompanying drawings in which:

FIG. 1 is a schematic diagram of a magnetic resonance imaging (MRI) system, according to an exemplary embodiment;
FIG. 2 is a block diagram of an MRI apparatus, according to an exemplary embodiment;
FIG. 3 illustrates an example in which a processor determines a first slice and a second slice;
FIG. 4 is a diagram for describing an operation, performed by the processor, of obtaining information about an RR interval of a heart, according to an exemplary embodiment;
FIG. 5 illustrates a first pulse sequence and a second pulse sequence, according to an exemplary embodiment;
FIG. 6 illustrates an example in which the processor applies a first pulse sequence to an object;
FIG. 7 illustrates an example in which the processor applies a second pulse sequence to an object;
FIG. 8 illustrates magnetization changes in an object, according to an exemplary embodiment;
FIG. 9 illustrates magnetization changes in an object, according to another exemplary embodiment;
FIG. 10 is a diagram for describing an operation, performed by the processor, of determining a first pulse sequence and a second pulse sequence, according to another exemplary embodiment;
FIG. 11 illustrates an example in which the processor applies a second pulse sequence to an object, according to another exemplary embodiment;

FIG. 12 illustrates magnetization changes in an object, according to another exemplary embodiment;

FIG. 13 is a flowchart of a method of operating the MRI apparatus, according to an exemplary embodiment;

FIG. 14 is a flowchart of a method, performed by the MRI apparatus, of acquiring a first MR signal based on a first pulse sequence, according to an exemplary embodiment;

FIG. 15 is a flowchart of a method, performed by the MRI apparatus, of applying a second pulse sequence to a second slice, according to an exemplary embodiment; and

FIG. 16 is a flowchart of a method, performed by the MRI apparatus, of applying a first pulse sequence and a second pulse sequence which use a long repetition time (TR) to an object, according to another exemplary embodiment.

## DETAILED DESCRIPTION

The present specification describes exemplary principles of the present disclosure and sets forth exemplary embodiments thereof to clarify the scope of the present disclosure and to allow those of ordinary skill in the art to implement the exemplary embodiments. The present exemplary embodiments may have different forms.

Like reference numerals generally refer to like elements throughout. The present specification does not describe all components in the exemplary embodiments, and common knowledge in the art or the same descriptions of the exemplary embodiments will be omitted below. In the specification, the term "part" or "portion" may be implemented as hardware or software, and according to exemplary embodiments, one "part" or "portion" may be formed as a single unit or element or include a plurality of units or elements. Hereinafter, the principles and exemplary embodiments of the present disclosure will be described in detail with reference to the accompanying drawings.

As used herein, the term "and/or" includes any and all combinations of one or more of the associated listed items. Expressions such as "at least one of," when preceding a list of elements, modify the entire list of elements and do not modify the individual elements of the list.

In the present disclosure, an "image" may include a medical image obtained by a magnetic resonance imaging (MRI) apparatus, a computed tomography (CT) apparatus, an ultrasound imaging apparatus, an X-ray apparatus, another medical imaging apparatus, or any other type of imaging apparatus, as desired.

Furthermore, in the present disclosure, an "object" may be a target to be imaged, and may include a human, an animal, or a part of a human or animal. For example, the object may include a body part, for example an organ, or a phantom.

In the present disclosure, a 'slice' may refer to a unit area of an object from which a magnetic resonance (MR) signal is to be acquired.

In exemplary embodiments, an MRI system acquires an MR signal and reconstructs the acquired MR signal into an image. The MR signal denotes a radio frequency (RF) signal emitted from the object.

In the MRI system, a main magnet generates a static magnetic field to align a magnetic dipole moment of a specific atomic nucleus of the object placed in the static magnetic field along a direction of the static magnetic field. A gradient coil may generate a gradient magnetic field by applying a gradient signal to a static magnetic field and induce resonance frequencies differently according to each region of the object. In other words, a resonance frequency induced in one region of the object may differ from a resonance frequency induced in a different region of the object.

An RF coil may emit an RF signal to match a resonance frequency of a region of the object of which an image is to be obtained. Furthermore, when gradient magnetic fields are applied, the RF coil may receive MR signals having different resonance frequencies emitted from a plurality of regions of the object. Though this process, the MRI system may obtain an image from an MR signal by using an image reconstruction technique.

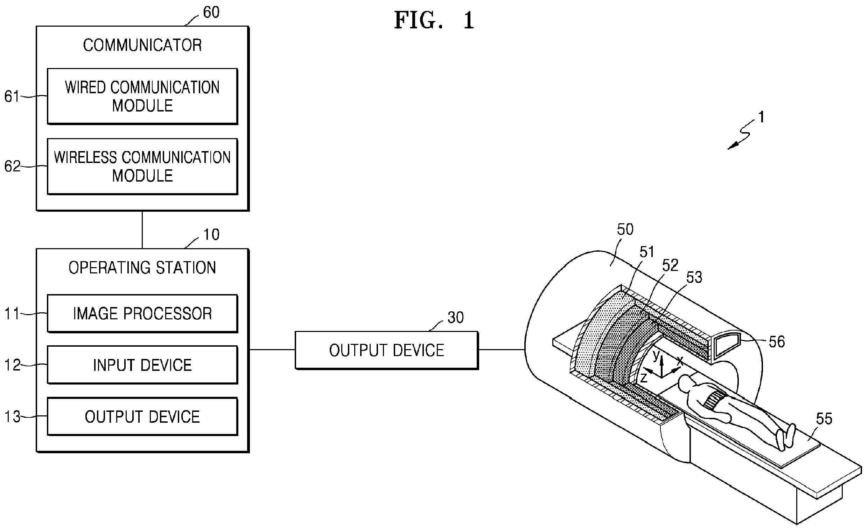

FIG. 1 is a schematic diagram of an MRI system 1, according to an exemplary embodiment. Referring to FIG. 1, the MRI system 1 may include an operating station 10, a controller 30, and a scanner 50. In exemplary embodiments, the controller 30 may be independently implemented as illustrated in FIG. 1. In exemplary embodiments, the controller 30 may be separated into a plurality of sub-components and may be incorporated into components in the MRI system 1. Operations of exemplary embodiments of the components in the MRI system 1 will now be described in detail.

The scanner 50 may have a cylindrical shape, for example, a shape of a bore, having an empty inner space into which an object may be inserted. A static magnetic field and a gradient magnetic field are generated in the inner space of the scanner 50, and an RF signal is emitted toward the inner space.

The scanner 50 may include a static magnetic field generator 51, a gradient magnetic field generator 52, an RF coil 53, a table 55, and a display 56. The static magnetic field generator 51 generates a static magnetic field for aligning magnetic dipole moments of atomic nuclei of the object in a direction of the static magnetic field. The static magnetic field generator 51 may be formed as a permanent magnet or superconducting magnet using a cooling coil.

The gradient magnetic field generator 52 is connected to the controller 30. The gradient magnetic field generator 52 generates a gradient magnetic field by applying a gradient to a static magnetic field in response to a control signal received from the controller 30. The gradient magnetic field generator 52 includes X, Y, and Z coils for generating gradient magnetic fields in X-, Y-, and Z-axis directions crossing each other at right angles and generates a gradient signal according to a position of a region being imaged in order to induce resonance frequencies differently according to regions of the object.

The RF coil 53 connected to the controller 30 may emit an RF signal toward the object in response to a control signal received from the controller 30 and may receive an MR signal emitted from the object. In detail, the RF coil 53 may transmit, toward atomic nuclei of the object having precessional motion, an RF signal having the same frequency as that of the precessional motion, may stop transmitting the RF signal, and then may receive an MR signal emitted from the object.

The RF coil 53 may be formed as a transmitting RF coil for generating an electromagnetic wave having an RF corresponding to the type of an atomic nucleus, a receiving RF coil for receiving an electromagnetic wave emitted from an atomic nucleus, or one transmitting/receiving RF coil serving both functions of the transmitting RF coil and receiving RF coil. Furthermore, in addition to the RF coil 53, a separate coil may be attached to the object. Examples of the separate coil may include a head coil, a spine coil, a torso coil, and a knee coil according to a region being imaged or to which the separate coil is attached.

The display 56 may be disposed outside or inside the scanner 50. The display 56 may also be controlled by the controller 30, thereby providing a user or the object with information related to medical imaging.

Furthermore, the scanner 50 may include an object monitoring information acquirer configured to obtain and transmit monitoring information about a state of the object. For example, the object monitoring information acquirer may obtain monitoring information related to the object from a camera for capturing images of a movement or position of the object, a respiration measurer for measuring the respiration of the object, an electrocardiogram (ECG) measurer for measuring the electrical activity of the heart of the object, or a temperature measurer for measuring a temperature of the object and may transmit the obtained monitoring information to the controller 30. The controller 30 may in turn control an operation of the scanner 50 based on the monitoring information about the object. Exemplary embodiments of operations of the controller 30 will now be described in more detail.

The controller 30 may control overall operations of the scanner 50.

The controller 30 may control a sequence of signals formed in the scanner 50. The controller 30 may control the gradient magnetic field generator 52 and the RF coil 53 according to a pulse sequence received from the operating station 10 or a designed pulse sequence.

In exemplary embodiments, a pulse sequence may include all pieces of information required to control the gradient magnetic field generator 52 and the RF coil 53. For example, the pulse sequence may include information about a strength, a duration, and application timing of a pulse signal applied to the gradient magnetic field generator 52.

The controller 30 may control a waveform generator for generating a gradient wave, for example an electrical pulse according to a pulse sequence, and a gradient amplifier for amplifying the generated electrical pulse and transmitting the same to the gradient magnetic field generator 52. Thus, the controller 30 may control formation of a gradient magnetic field by the gradient magnetic field generator 52.

The controller 30 may control an operation of the RF coil 53. For example, the controller 30 may supply an RF pulse having a resonance frequency to the RF coil 53 that emits an RF signal toward the object, and may receive an MR signal received by the RF coil 53. In this case, the controller 30 may adjust emission of an RF signal and reception of an MR signal according to an operating mode by controlling an operation of a switch, for example a transmit/recieve (T/R) switch, for adjusting transmitting and receiving directions of the RF signal and the MR signal based on a control signal.

The controller 30 may control a movement of the table 55 on which the object is placed. Before MRI is performed, the controller 30 may move the table 55 according to which region of the object is to be imaged.

The controller 30 may also control the display 56. For example, the controller 30 may control the on/off state of the display 56 or a screen image to be output on the display 56 according to a control signal.

The controller 30 may an algorithm for controlling operations of the components in the MRI system 1, a memory for storing data in the form of a program, and a processor for performing the above-described operations by using the data stored in the memory. In some exemplary embodiments, the memory and the processor may be implemented as separate chips. In some exemplary embodiments, the memory and processor may be incorporated into a single chip.

The operating station 10 may control overall operations of the MRI system 1. The operating station 10 may include an image processor 11, an input device 12, and an output device 13.

The image processor 11 may control the memory to store an MR signal received from the controller 30, and may generate image data with respect to the object from the stored MR signal by applying an image reconstruction technique by using an image processor.

For example, if a k-space, also referred to as, for example, a Fourier space or a frequency space, of the memory is filled with digital data to complete $k$-space data, the image processor 11 may reconstruct image data from the $k$-space data by applying various image reconstruction techniques, for example, by performing inverse Fourier transform on the $k$-space data, by using the image processor.

Furthermore, the image processor 11 may perform various signal processing operations on MR signals in

5

parallel. For example, the image processor 11 may perform signal processing on a plurality of MR signals received via a multi-channel RF coil in parallel in order to convert the plurality MR signals into image data. In addition, the image processor 11 may store the image data in the memory, or the controller 30 may store the same in an external server using a communicator 60 as will be described below.

The input device 12 may receive, from the user, a control command for controlling the overall operations of the MRI system 1. For example, the input device 12 may receive, from the user, object information, parameter information, a scan condition, and information about a pulse sequence. The input device 12 may be a keyboard, a mouse, a trackball, a voice recognizer, a gesture recognizer, a touch screen, or any other input device.

The output device 13 may output image data generated by the image processor 11. The output device 13 may also output a user interface (UI) configured so that the user may input a control command related to the MRI system 1. The output device 13 may be formed as a speaker, a printer, a display, or the like.

Although FIG. 1 illustrates the operating station 10 and the controller 30 as separate components, in exemplary embodiments the operating station 10 and the controller 30 may be included in a single device as described above. In addition, processes respectively performed by the operating station 10 and the controller 30 may be performed by another component. For example, the image processor 11 may convert an MR signal received from the controller 30 into a digital signal, or the controller 30 may directly perform the conversion of the MR signal into the digital signal.

The MRI system 1 may further include the communicator 60 and may be connected to an external device such as a server, a medical apparatus, and a portable device , for example, a smartphone, a tablet personal computer (PC), a wearable device, etc., via the communicator 60.

The communicator 60 may include at least one component that enables communication with the external device. For example, the communicator 60 may include at least one of a short-range communication module, a wired communication module 61, and a wireless communication module 62.

The communicator 60 may receive a control signal and data from the external device and may transmit the received control signal to the controller 30 so that the controller 30 may control the MRI system 1 based on the received signal.

In exemplary embodiments, by transmitting a control signal to the external device via the communicator 60, the controller 30 may control the external device based on the control signal.

For example, the external device may process data of the external device based on the control signal received from the controller 30 via the communicator 60.

A program for controlling the MRI system 1 may be installed on the external device and may include instructions for performing some or all of the operations of the controller 30.

In exemplary embodiments, the program may be preinstalled on the external device, or a user of the external device may download the program from a server providing an application for installation. The server providing an application may include a recording medium having the program recorded thereon.

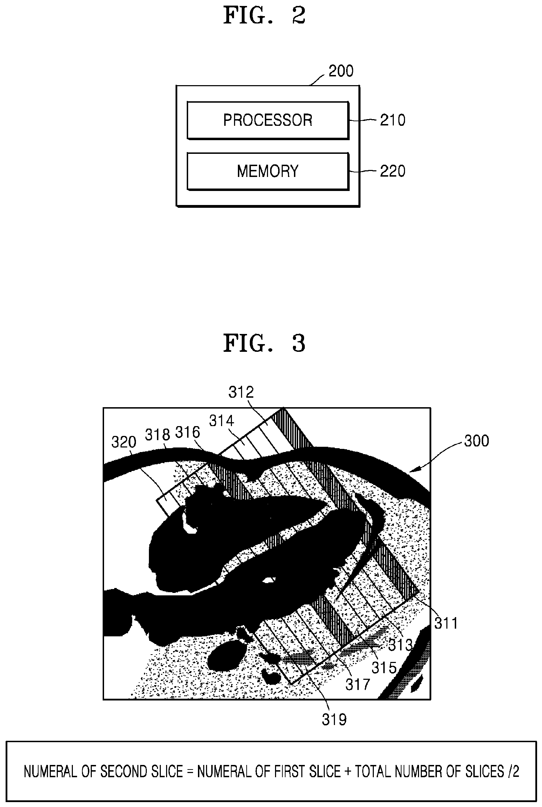

FIG. 2 is a block diagram of an MRI apparatus 200, according to an exemplary embodiment.

The MRI apparatus 200 illustrated in FIG. 2 may be any apparatus capable of imaging, processing, or reconstructing an MR image. For example, the MRI apparatus 200 may be an apparatus configured to apply RF pulses to the object via a plurality of channel coils in a scanner and to acquire MR signals obtained via the plurality of channel coils.

For example, the MRI apparatus 200 may be included in the MRI system 1 described with reference to FIG. 1. When the MRI apparatus 200 is included in the MRI system 1 of FIG. 1, a processor 210 of FIG. 2 may correspond to the operating station 10, or the controller 30, of FIG. 1. The scanner may correspond to the scanner 50 of FIG. 1.

The MRI apparatus 200 may be a server apparatus configured to provide a pulse sequence to be applied to the object, to receive an MR signal acquired by MRI scanning the object, and to reconstruct an MR image by using the received MR signal. For example, the MRI apparatus 200 may be a server, medical equipment, or a mobile phone configured to communicate with the communicator 60 of the MRI system 1 described with reference to FIG. 1, and may perform a reconstruction operation on the MR image by receiving the MR signal obtained from the MRI system 1.

Referring to FIG. 2, the MRI apparatus 200 may include the processor 210 and a memory 220.

The processor 210 may control general operations of the MRI apparatus 200, may determine a pulse sequence to be applied to the object during a scan time, and may reconstruct an MR image by using an MR signal acquired during the scan time.

According to the present exemplary embodiment, the processor 210 may apply, to the object, a first pulse sequence and a second pulse sequence using different repetition times (TRs) and echo times (TEs) during the scan time, and thus may reconstruct a plurality of MR images having different contrasts. In this regard, to apply a pulse sequence to the object may include an operation of transmitting, by the processor 210, the pulse sequence to be applied to the object to the scanner. In exemplary embodiments, applying a pulse sequence to the object may include an operation of controlling, by the processor 210, the scanner, based on the pulse sequence.

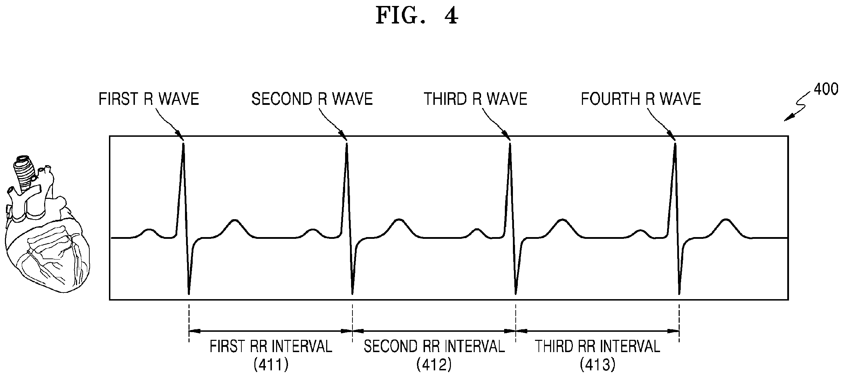

In exemplary embodiments, the processor 210 may scan a heart or a region around the heart. In this case, the object may move in response to, or during heartbeats. Thus, the processor 210 may determine a timing at which the first pulse sequence and the second pulse sequence are applied to the object, by using an RR interval, which may be an interval between an R wavelength and a next R wavelength, of the heart. For example, the processor 210 may apply the first pulse sequence to the object during a first RR interval, and may apply the second pulse sequence to the object during a second RR interval. An exemplary embodiment of a method of obtaining, by the processor 210, information about the RR interval of the heart will be described below with reference to FIG. 4.

In addition, the processor 210 may determine, or generate, the first pulse sequence and the second pulse sequence. For example, the processor 210 may determine the first pulse sequence using a long TR and a long TE and the second pulse sequence using a short TE.

In exemplary embodiments, the processor 210 may determine the first pulse sequence by using a black-blood imaging technique that suppresses a blood signal of the object. The first pulse sequence may use at least two RR intervals as one TR. In addition, in order to increase a difference between signals of tissues, for example, walls of the heart, in the object, the processor 210 may determine the first pulse sequence using the long TE.

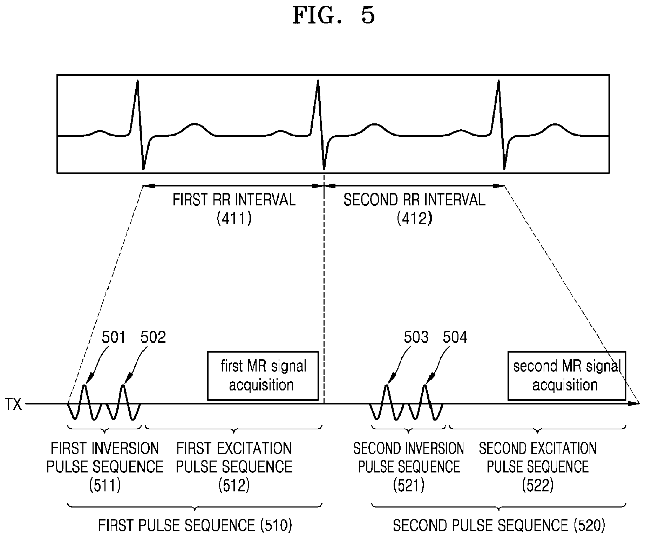

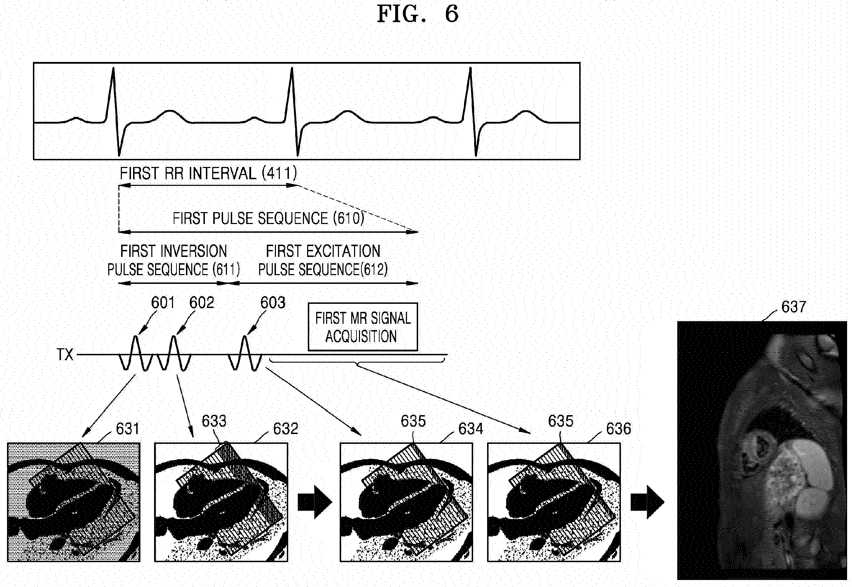

The processor 210 may determine the first pulse sequence such that the first pulse sequence further suppresses a fat signal. An exemplary embodiment of a method of determining, by the processor 210, the first pulse sequence will be described in detail with reference to FIGS. 5 and 6.

Furthermore, in order to increase the difference between the signals of the tissues in the object, the processor 210 may determine the second pulse sequence using a short TE. An exemplary embodiment of a method of determining, by the processor 210, the second pulse sequence will be described below with reference to FIGS. 5 and 6.

Because the first pulse sequence uses a long TR, for example, a period of two RR intervals, when the first pulse sequence is applied to the object during a first RR interval, atomic nuclei in the object which are excited by the first pulse sequence may need to be relaxed during a second RR interval. Thus, the processor 210 may control the second pulse sequence to be applied to a position of the object which is different from a position to which the first pulse sequence is applied.

To do so, the processor 210 may apply the first pulse sequence to a first slice from among a plurality of slices corresponding to an imaging-target area in the object, for example, the heart or a region around the heart, during the first RR interval. The processor 210 may apply the second pulse sequence to a second slice that is discontinuously separate from a position of the first slice during the second RR interval. Because the second pulse sequence is applied to the second slice that is discontinuous with respect to the first slice, atomic nuclei in the first slice excited by the first pulse sequence may be relaxed during the second RR interval.

When the first pulse sequence is applied to the first slice, the processor 210 may acquire a first MR signal corresponding to the first slice. The processor 210 may reconstruct a first MR image corresponding to the first slice, based on the first MR signal. In this regard, the first MR image may be a T2-weighted image according to the long TR and the long TE. Because the first MR image has high contrast between a blood flow and a tissue, for example, the walls of the heart, the first MR image may be used to recognize a lesion in the object, for example, the heart or a region around the heart.

Furthermore, because the second pulse sequence is applied to the second slice, the processor 210 may acquire a second MR signal corresponding to the second slice. The processor 210 may reconstruct a second MR image corresponding to the second slice, based on the second MR signal. In this regard, the second MR image may be a T1-weighted image according to a short TR and a short TE. Because the second MR image has high contrast between tissues, the second MR image may be used to recognize anatomy information of the object.

The processor 210 may reconstruct an MR image by using various techniques. For example, the processor 210 may reconstruct the MR image by using the Sensitivity Encoding (SENSE) technique, the GeneRalized Autocalibrating Partially Parallel Acquisitions (GRAPPA) technique, or the like.

The memory 220 may be connected to the processor 210 in a wired or wireless manner, and may temporarily or permanently store information processed by the processor 210. According to the present exemplary embodiment, the memory 220 may store the first MR signal and the second MR signal acquired by the processor 210. In addition, the memory 220 may store the first MR image and the second MR image reconstructed by the processor 210.

Exemplary embodiments of operations of the MRI apparatus 200 during the first RR interval and the second RR interval are described above, but the MRI apparatus 200 may repeatedly perform the operations with respect to third through $N^{th}$ RR intervals. For example, during the third through $N^{th}$ RR intervals, the processor 210 may alternately perform the operation corresponding to the first RR interval and the operation corresponding to the second RR interval, may reconstruct a first MR image by using MR signals acquired in odd RR intervals, and may reconstruct a second MR image by using MR signals acquired in even RR intervals.

In this manner, the MRI apparatus 200 according to exemplary embodiments reconstructs a plurality of MR images including different items of information during a scan time, thereby improving operating efficiency.

FIG. 3 shows an example in which the processor 210 determines, or selects, a first slice and a second slice. For convenience of description, FIG. 3 illustrates ten slices 311 through 320. However, the number of slices is not limited to ten.

Referring to FIG. 3, the processor 210 may determine a first slice 311 and a second slice 316 from among the slices 311 through 320 corresponding to an MR imaging target area in an object 300, wherein the second slice 316 is separate from a position of the first slice 311 and is discontinuous with respect to the first slice 311. The processor 210 may determine the second slice 316 by using numerals allocated to the slices 311 through 320.

In the present exemplary embodiment, the processor 210 may determine the second slice 316 by using Equation 1.

[Equation 1]

$$\text{Numeral of second slice} = \text{numeral of first slice} + \text{total number of slices}/2$$

For example, the processor 210 may determine the numeral of the second slice 316 in a manner that the processor 210 adds the numeral of the first slice 311 to a result value obtained by dividing the number of all slices 311 through 320 by a preset number, for example '2'. As illustrated in FIG. 3, when the numeral of the first slice 311 is 1, the processor 210 may determine the numeral of the second slice 316 to be 6.

FIG. 4 is a diagram for describing an operation, performed by the processor 210, of obtaining information about an RR interval of a heart, according to an exemplary embodiment.

Referring to FIG. 4, the processor 210 may obtain heartbeat information 400 of a patient via an ECG measurer placed inside or outside the MRI apparatus 200. In exemplary embodiments, processor 210 may obtain the heartbeat information 400 of the patient via a photoplethysmogram (PPG) device worn on a finger of the patient.

The processor 210 may analyze the heartbeat information 400, thereby recognizing R waves of a heart. The processor 210 may determine an interval between a first R wave and a second R wave to be a first RR interval 411. In addition, the processor 210 may determine an interval between the second R wave and a third R wave to be a second RR interval 412.

Before scanning is performed on an object, the processor 210 may analyze the heartbeat information 400 of the patient and may calculate a time interval between two sequential R waves. In addition, the processor 210 may determine the number of RR intervals corresponding to a TR of a first pulse sequence by using the calculated time interval. For example, if the calculated time interval is 700 ms, and the TR of the first pulse sequence is 1400 ms, the processor 210 may determine that the TR of the first pulse sequence corresponds to two RR intervals. As another example, if the calculated time interval is 500 ms, and the TR of the first pulse sequence is 1400 ms, the processor 210 may determine that the TR of the first pulse sequence corresponds to three RR intervals. The processor 210 may apply the first pulse sequence to a first slice during the first RR interval 411, and may sequentially apply the second pulse sequence to a second slice during the second RR interval 412 and a third RR interval 413.

FIG. 5 illustrates a first pulse sequence 510 and a second pulse sequence 520, according to an exemplary embodiment.

Referring to FIG. 5, the processor 210 may determine the first pulse sequence 510 to be applied to a first slice during the first RR interval 411, and may determine the second pulse sequence 520 to be applied to a second slice during the second RR interval 412.

According to the present exemplary embodiment, the first pulse sequence 510 may include a first inversion pulse sequence 511 for suppressing a blood signal of an object, and a first excitation pulse sequence 512 for acquiring a first MR signal corresponding to the first slice.

In exemplary embodiments, the first inversion pulse sequence 511 may include a first inversion RF pulse 501 and a second inversion RF pulse 502. The first inversion RF pulse 501 may invert magnetization, for example, longitudinal magnetization in atomic nuclei, of the object, and the second inversion RF pulse 502 may recover the magnetization inverted by the first inversion RF pulse 501.

The processor 210 may apply the first inversion RF pulse 501 to the entire object, based on the first inversion pulse sequence 511, and may apply the second inversion RF pulse 502 to the first slice. For example, when the second inversion RF pulse 502 is applied to the object, the processor 210 may generate a space encoding gradient magnetic field corresponding to the first slice in the scanner, and thus may apply the second inversion RF pulse 502 to the first slice. When the second inversion RF pulse 502 is applied to the first slice, the processor 210 may recover magnetization in the first slice to an original state. An inversion RF pulse may be, for example, a 180° RF pulse.

Then, the processor 210 may apply the first excitation pulse sequence 512 to the first slice in which the magnetization is recovered. The first excitation pulse sequence 512 may include at least one excitation RF pulse. For example, the processor 210 may determine the first excitation pulse sequence 512 including at least one excitation RF pulse, by using a spin-echo (SE) technique, a fast spin-echo (FSE) technique, or the like. However, the present disclosure is not limited thereto, and the first excitation pulse sequence 512 may be determined by using other techniques as desired. The excitation RF pulse may be, for example, a 90° RF pulse.

When at least one excitation RF pulse is applied to the object, the processor 210 may generate the space encoding gradient magnetic field corresponding to the first slice in the scanner, and thus may apply the first excitation pulse sequence 512 to the first slice.

The processor 210 may apply the first excitation pulse sequence 512 by using an inversion time (TI) of, for example, blood. In more detail, blood in the first slice in which the magnetization is recovered by the second inversion RF pulse 502 flows through blood vessels to another position in the object. Thus, blood in which magnetization was inverted by the first inversion RF pulse 501 may be present in the first slice. The processor 210 may predict, by using a TI of the blood, a time when an intensity of an MR signal is decreased from the blood in which the magnetization was inverted by the first inversion RF pulse 501, and may apply the first excitation pulse sequence 512 to the first slice by using the predicted time. Thus, the first MR signal acquired by the first excitation pulse sequence 512 may correspond to the first slice in which a blood signal is suppressed.

The first excitation pulse sequence 512 may further include a pulse sequence for suppressing, for example, a fat signal of the object. For example, the processor 210 may apply the first excitation pulse sequence 512 including at least one inversion RF pulse and at least one excitation RF pulse to the first slice by using a short TI inversion recovery (STIR) technique. In this case, the processor 210 may acquire the first MR signal in which a fat signal in the first slice is suppressed, by using TI of fat.

According to the present exemplary embodiment, the second pulse sequence 520 may include a second inversion pulse sequence 521 for suppressing a blood signal of the object, and a second excitation pulse sequence 522 for acquiring a second MR signal corresponding to the second slice.

For example, the second inversion pulse sequence 521 may include a third inversion RF pulse 503 and a fourth inversion RF pulse 504. The third inversion RF pulse 503 may invert magnetization in the object in an opposite direction, and the fourth inversion RF pulse 504 may recover the magnetization inverted by the third inversion RF pulse 503. The processor 210 may apply the third inversion RF pulse 503 to the entire object, based on the second inversion pulse sequence 521, and may apply the fourth inversion RF pulse 504 to the first slice and the second slice. In this regard, because the third inversion RF pulse 503 is applied to the entire object, the third inversion RF pulse 503 may influence the first slice. Thus, when the fourth inversion RF pulse 504 is applied to the object, the processor 210 may generate not only a space encoding gradient magnetic field corresponding to the second slice but may also generate a space encoding gradient magnetic field corresponding to the first slice, thereby restoring magnetization in the first slice and the second slice to original states.

In exemplary embodiments, the processor 210 may apply the second excitation pulse sequence 522 to the second slice in which the magnetization is recovered. For example, the processor 210 may generate the space encoding gradient magnetic field corresponding to the second slice, with respect to at least one excitation RF pulse.

The second excitation pulse sequence 522 may include, but is not limited to, at least one excitation RF pulse based on the SE technique, the FSE technique, or the like.

The processor 210 may apply the second inversion pulse sequence 521 by using TI of blood, and may acquire the second MR signal in which a blood signal in the second slice is suppressed.

FIG. 6 illustrates an example in which the processor 210 applies a first pulse sequence 610 to an object.

Referring to FIG. 6, the processor 210 may control first inversion RF pulse 601, second inversion RF pulse 602, and third inversion RF pulse 603, and at least one excitation RF pulse, which are included in the first pulse sequence 610, to influence all regions or specific region of the object.

In the exemplary embodiment illustrated in image 631 of FIG. 6, the first inversion RF pulse 601 may be applied to all regions of the object. The processor 210 may not generate a space encoding gradient magnetic field corresponding to the first inversion RF pulse 601, thereby allowing the first inversion RF pulse 601 to be applied to all regions of the object.

In addition, as illustrated in image 632, the processor 210 may apply the second inversion RF pulse 602 to a first region 633 including a first slice 635. In the present exemplary embodiment, the first region 633 may include, but is not limited to, two or more slices. When the second inversion RF pulse 602 is applied to the object, the processor 210 may generate a space encoding gradient magnetic field corresponding to the first region 633. In this regard, a thickness of the first region 633 may be, for example, at least two times higher than a thickness of the first slice 635.

In exemplary embodiments, the processor 210 may apply, to the first slice 635, a first excitation pulse sequence 612 based on the STIR technique. When a third inversion RF pulse 603 is applied to the object, the processor 210 may generate a space encoding gradient magnetic field corresponding to the first slice 635, as illustrated in an image 634.

When the at least one excitation RF pulse is applied to the object, the processor 210 may generate a space encoding gradient magnetic field corresponding to the first slice 635, as illustrated in an image 636.

The processor 210 may determine timing to apply the at least one excitation RF pulse included in the first excitation pulse sequence 612 to the object by using, for example, the TI of fat. For example, the processor 210 may apply an excitation RF pulse to the object 160 ms after the third inversion RF pulse 603 is applied to the object, thereby acquiring a first MR signal in which a blood signal and a fat signal are removed.

An image 637 of FIG. 6 illustrates an example of a first MR image reconstructed by using the first MR signal acquired by the processor 210. In the example, the first MR image may be a T2-weighted image in which the blood signal and the fat signal are suppressed.

In the above descriptions, an operation, performed by the processor 210, of generating a space encoding gradient magnetic field may include an operation, performed by the processor 210, of providing a control signal to the scanner placed inside or outside the MRI apparatus 200.

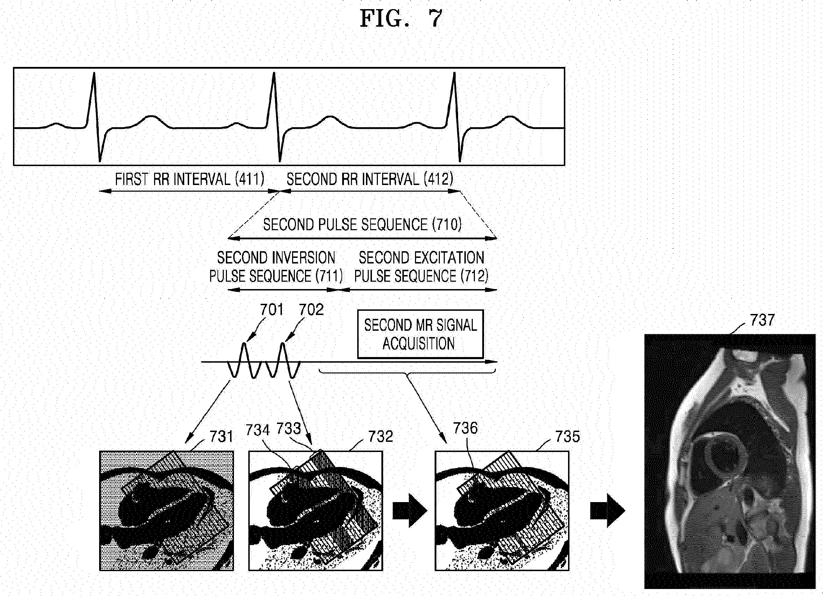

FIG. 7 illustrates an example in which the processor 210 applies a second pulse sequence 710 to an object.

Referring to FIG. 7, the processor 210 may control first inversion RF pulse 701 and second inversion RF pulse 702, and at least one excitation RF pulse, which are included in the second pulse sequence 710, to influence all regions or specific region of the object.

As illustrated in image 731 of FIG. 7, the first inversion RF pulse 701 may be applied to all regions of the object. The processor 210 may not generate a space encoding gradient magnetic field corresponding to the first inversion RF pulse 701, thereby allowing the first inversion RF pulse 701 to be applied to all regions of the object.

As illustrated in an image 732, the processor 210 may apply the second inversion RF pulse 702 to a first region 733 including a first slice, which may correspond to the first slice 635 of FIG. 6, and a second region 734 including a second slice 736. In the present exemplary embodiment, each of the first region 733 and the second region 734 may include two or more slices, but the present disclosure is not limited thereto. When the second inversion RF pulse 702 is applied to the object, the processor 210 may generate space encoding gradient magnetic fields corresponding to the first region 733 and the second region 734. In this regard, the processor 210 may generate the space encoding gradient magnetic field corresponding to the first region 733 in order to minimize an influence of the first inversion RF pulse 701 on the first slice 635. Respective thicknesses of the first region 733 and the second region 734 may be, for example, at least two times higher than respective thicknesses of the first slice 635 and the second slice 736.

In exemplary embodiments, the processor 210 may apply a second excitation pulse sequence 712 for acquiring a second MR signal to the second slice 736. When at least one excitation RF pulse included in the second excitation pulse sequence 712 is applied to the object, the processor 210 may generate the space encoding gradient magnetic field corresponding to the second slice 736 as illustrated in an image 735.

Image 737 of FIG. 7 illustrates an example of a second MR image reconstructed by using the second MR signal acquired by the processor 210.

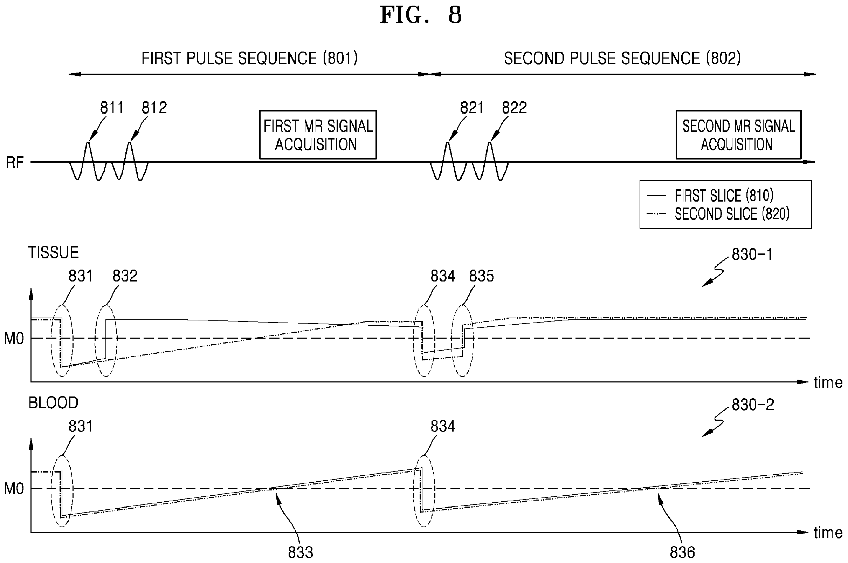

FIG. 8 illustrates magnetization changes in an object, according to an exemplary embodiment.

Referring to FIG. 8, the processor 210 may acquire a first MR signal and a second MR signal using magnetization changes 830-1 and 830-2 in tissues and blood in the object, the magnetization changes 830-1 and 830-2 occurring as a result of a first pulse sequence 801 and a second pulse sequence 802.

In exemplary embodiments, when a first inversion RF pulse 811 included in the first pulse sequence 801 is applied to all regions of the object, as illustrated at mark 831, magnetization in the tissues and the blood in the object may be inverted. Afterward, when a second inversion RF pulse 812 included in the first pulse sequence 801 is applied to a first slice 810, as illustrated in a mark 832, magnetization in the first slice 810 may be recovered.

The processor 210 may apply at least one excitation RF pulse to the first slice 810 by using TI of blood. Thus, the processor 210 may acquire the first MR signal at a first time 833 when magnetization in the blood which was inverted by the first inversion RF pulse 811 approaches '0'. In the present exemplary embodiment, the processor 210 may acquire the first MR signal by selecting a long TE.

Continuously, when a third inversion RF pulse 821 included in the second pulse sequence 802 is applied to all regions of the object during a second RR interval, as illustrated at mark 834, magnetization in the tissues and the blood in the object may be inverted again. Afterward, when a fourth inversion RF pulse 822 included in the second pulse sequence 802 is applied to the first slice 810 and a second slice 820, as illustrated in a mark 835, magnetization in the first slice 810 and the second slice 820 may be recovered.

During the second RR interval, the processor 210 may apply at least one excitation RF pulse to the second slice 820 by using TI of blood. Thus, the processor 210 may acquire the second MR signal at a second time 836 when magnetization in the blood which was inverted by the third inversion RF pulse 821 approaches '0'. In the present exemplary embodiment, the processor 210 may acquire the second MR signal by selecting a short TE.

In this manner, the MRI apparatus 200 according to the present exemplary embodiment may apply a pulse sequence to discontinuous slices at every RR interval, and thus may acquire MR signals, so that an efficiency of the MRI apparatus 200 may be improved.

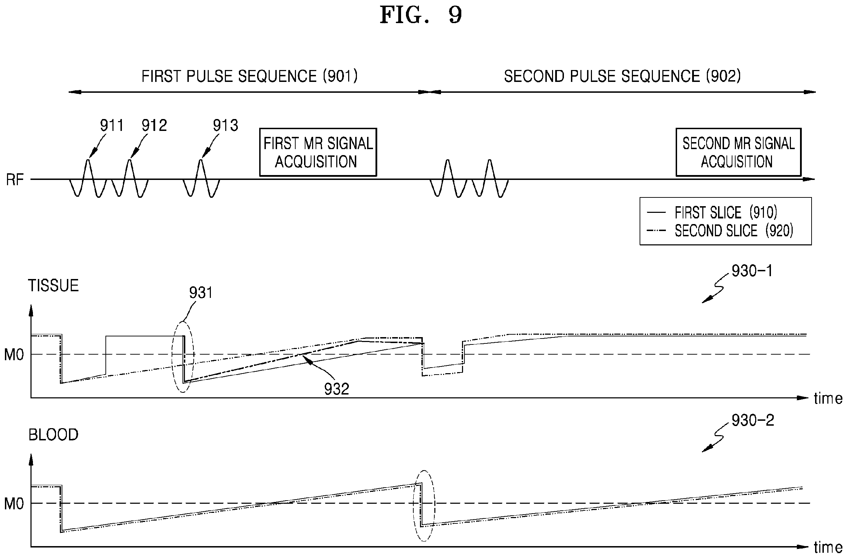

FIG. 9 illustrates magnetization changes in an object, according to another exemplary embodiment.

Referring to FIG. 9, the processor 210 may apply a first pulse sequence 901 for suppressing a fat signal in the object to a first slice 910.

In exemplary embodiments, the processor 210 may apply a first inversion RF pulse 911 and a second inversion RF pulse 912 included in the first pulse sequence 901 to the first slice 910 and may apply, to the first slice 910, a third inversion RF pulse 913 for inverting magnetization in fat among components forming a tissue. As illustrated at mark 931, magnetization in the first slice 910 may be inverted by the third inversion RF pulse 913. In this regard, a change in magnetization in blood in the first slice 910 may be diluted due to blood flowing from another slice.

The processor 210 may acquire a first MR signal by using TI of fat. In exemplary embodiments, the processor 210 may acquire the first MR signal at a first time 932 when the magnetization in fat which was inverted by the third inversion RF pulse 913 approaches '0'. Thus, the processor 210 may reconstruct a first MR image by using the first MR signal in which a fat signal is suppressed. Magnetization in blood which was inverted by the first inversion RF pulse 911 may approach '0' at the first time 932.

Continuously, the processor 210 may apply a second pulse sequence 902 to a second slice 920 during a second RR interval, thereby reconstructing a second MR image.

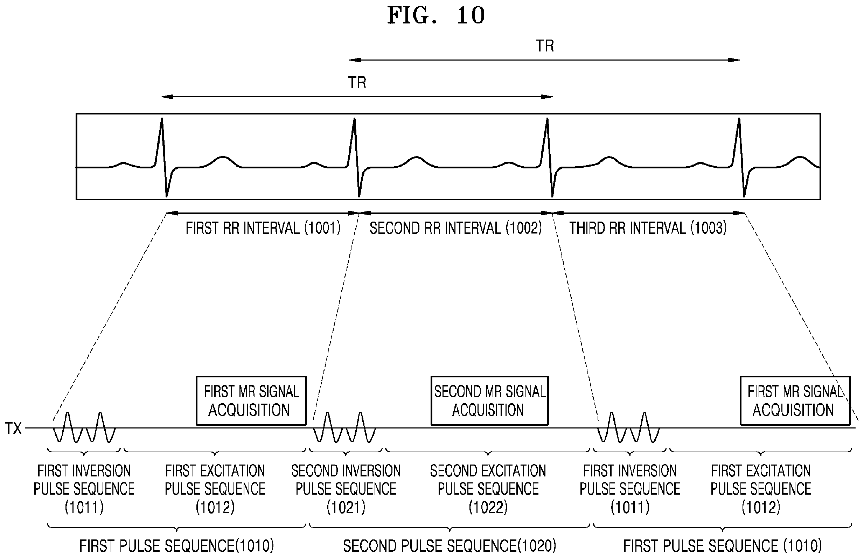

FIG. 10 is a diagram for describing an operation, performed by the processor 210, of determining a first pulse sequence and a second pulse sequence, according to another exemplary embodiment. According to the present exemplary embodiment, the processor 210 may determine the first pulse sequence using a long TR and a long TE and the second pulse sequence using a long TR and a short TE. For example, the processor 210 may determine the second pulse sequence such that the second pulse sequence uses two RR intervals as one TR.

Referring to FIG. 10, the processor 210 may determine a first pulse sequence 1010 that uses two RR intervals as one TR, and a second pulse sequence 1020 that uses two RR intervals as one TR.

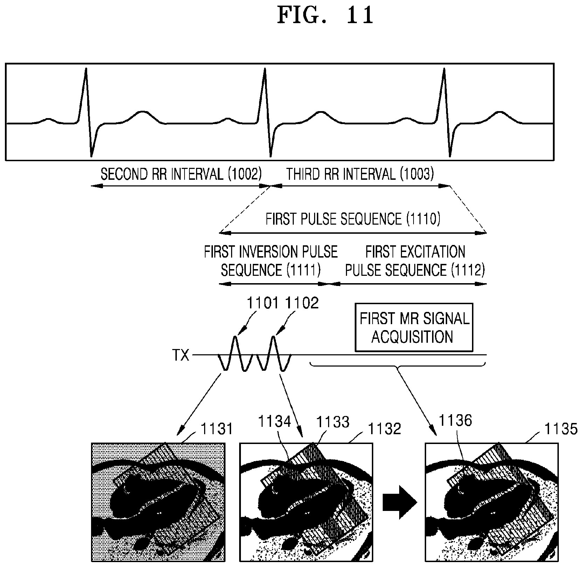

Thus, atomic nuclei in a second slice excited by a second excitation pulse sequence 1022 of the second pulse sequence 1020 are required to be relaxed during a third RR interval 1003. The processor 210 may perform an operation of minimizing an influence of the first pulse sequence 1010 on the second slice during the third RR interval 1003. An exemplary embodiment of an operation performed by the processor 210 during the third RR interval 1003 will be described below with reference to FIG. 11.

Because the processor 210 acquires a second MR signal using a short TE, a second MR image reconstructed from the second MR signal may be a proton density (PD)-weighted image.

FIG. 11 illustrates an example in which the processor 210 applies a second pulse sequence to an object, according to another exemplary embodiment.

Referring to FIG. 11, in order to minimize an influence applied to a second slice by a first pulse sequence 1110 during a third RR interval 1003, the processor 210 may vary regions of the object to which a second inversion RF pulse 1102 included in the first pulse sequence 1110 is to be applied.

In exemplary embodiments, after a first inversion RF pulse 1101 is applied to all regions of the object, for example as in image 1131, when the second inversion RF pulse 1102 is applied to the object, as illustrated in image 1132, the processor 210 may generate not only a space encoding gradient magnetic field corresponding to a first region 1133 including a first slice 1136 but may also generate a space encoding gradient magnetic field corresponding to a second region 1134 including a second slice. Thus, the processor 210 may recover magnetization in the second slice which was inverted by the first inversion RF pulse 1101.

Afterward, as illustrated in image 1135, the processor 210 may apply a first excitation pulse sequence 1112 to the first slice 1136, thereby acquiring a first MR signal.

In the above descriptions, for convenience of description, an operation that is performed by the processor 210 during the third RR interval 1003 is described but the operation performed by the processor 210 may also be performed during a first RR interval.

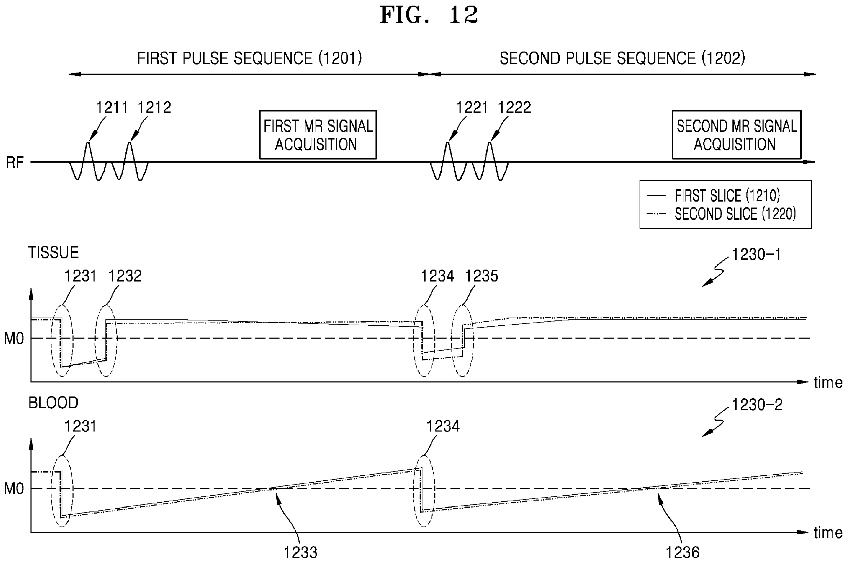

FIG. 12 illustrates magnetization changes in an object, according to another exemplary embodiment.

Referring to FIG. 12, the processor 210 may acquire a first MR signal and a second MR signal by using magnetization changes 1230-1 and 1230-2 in tissues and blood in the object, the magnetization changes 1230-1 and 1230-2 occurring as a result of a first pulse sequence 1201 and a second pulse sequence 1202.

In exemplary embodiments, when a first inversion RF pulse 1211 included in the first pulse sequence 1201 is applied to all regions of the object, as illustrated in a mark 1231, magnetization in the tissues and the blood in the object may be inverted. Afterward, when a second inversion RF pulse 1212 included in the first pulse sequence 1201 is applied to a first slice 1210 and a second slice 1220, as illustrated at mark 1232, magnetization in tissues of the first slice 1210 and the second slice 1220 may be recovered.

The processor 210 may apply at least one excitation RF pulse by using TI of blood, thereby acquiring the first MR signal at a first time 1233 when magnetization in blood approaches '0'. In this regard, the processor 210 may select a long TE.

Continuously, when a third inversion RF pulse 1221 included in the second pulse sequence 1202 is applied to all regions of the object during a second RR interval, as illustrated at mark 1234, magnetization in the first slice 1210 and the second slice 1220 may be inverted again. Afterward, when a fourth inversion RF pulse 1222 included in the second pulse sequence 1202 is applied to the first slice 1210 and the second slice 1220, as illustrated at mark 1235, magnetization in the first slice 1210 and the second slice 1220 may be recovered.

During the second RR interval, the processor 210 may acquire, by using TI of blood, the second MR signal at a second time 1236 when magnetization in blood which was inverted by the third inversion RF pulse 1221 approaches '0'. In this regard, the processor 210 may select a short TE. Accordingly, the processor 210 may reconstruct a PD-weighted image from the second MR signal.

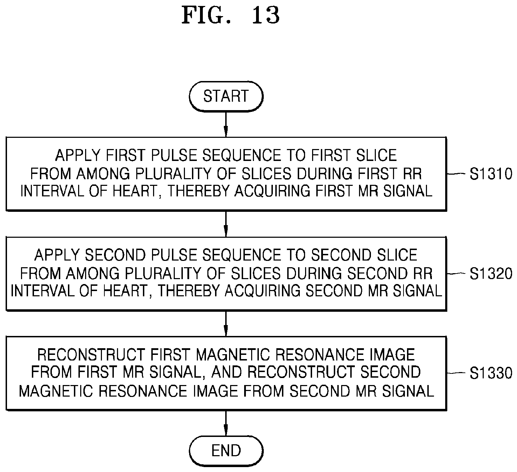

FIGS. 13 through 16 are flowcharts for describing a method of operating the MRI apparatus 200, according to exemplary embodiments. The method of operating the MRI apparatus 200 described with reference to FIGS. 13 through 16 is related to the exemplary embodiments described with reference to FIGS. 1 through 12. Thus, the descriptions above with reference to FIGS. 1 to 12 may also generally be applied to the flowcharts of FIGS. 13 to 16.

FIG. 13 is a flowchart of a method of operating the MRI apparatus 200, according to an exemplary embodiment.

Referring to FIG. 13, in operation S1310, the MRI apparatus 200 may apply a first pulse sequence to a first slice from among a plurality of slices during a first RR interval of a heart, thereby acquiring a first MR signal. In order to scan the heart or a region around the heart, the MRI apparatus 200 may recognize an RR interval, which may be an interval between an R wavelength and a next R wavelength of the heart, and may apply the first pulse sequence to the first slice by using the recognized RR interval.

According to the present exemplary embodiment, the MRI apparatus 200 may determine the first pulse sequence to use a long TR and a long TE. For example, the first pulse sequence may use at least two RR intervals as one TR.

In addition, the MRI apparatus 200 may apply the first pulse sequence for suppressing a blood signal in the first slice to the first slice, by using a black-blood imaging technique. In the present exemplary embodiment, the first pulse sequence may include a first inversion pulse sequence for suppressing a blood signal and a first excitation pulse sequence for acquiring a first MR signal.

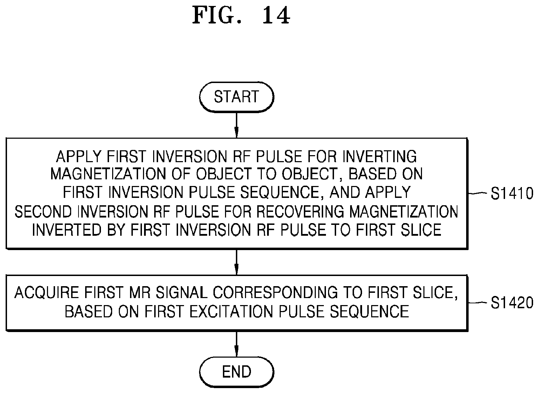

Also, the MRI apparatus 200 may apply the first pulse sequence for suppressing a fat signal among components forming a tissue to the first slice, by using a STIR technique for suppressing a fat signal. In this case, the processor 210 may further add an RF pulse for suppressing a fat signal to the first excitation pulse sequence. The first pulse sequence to be applied to the first slice by the MRI apparatus 200 will be described below with reference to FIG. 14.

When the first pulse sequence is applied to the first slice, the MRI apparatus 200 may acquire the first MR signal corresponding to the first slice. In this regard, the first MR signal may have a large signal difference between blood and a tissue, due to the use of a long TE.

In operation S1320, the MRI apparatus 200 may apply a second pulse sequence to a second slice from among the slices during a second RR interval of the heart, thereby acquiring a second MR signal. The second slice may be discontinuously separate from a position of the first slice during the second RR interval of the heart.

According to the present exemplary embodiment, the MRI apparatus 200 may allocate numerals to the slices, and may determine the second slice by using a numeral of the first slice and the number of the slices, the second slice being discontinuous with respect to the first slice. For example, the MRI apparatus 200 may determine the second slice in a manner that the MRI apparatus 200 adds the numeral of the first slice to a result value obtained by dividing the number of the slices by 2. Because the first pulse sequence uses the long TR, an influence of the second pulse sequence on the first slice during the second RR interval may be minimized.

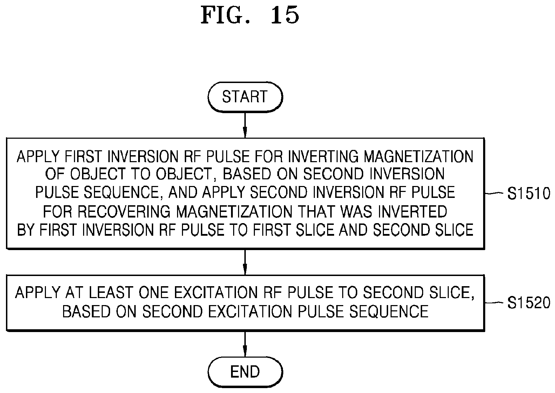

According to the present exemplary embodiment, the MRI apparatus 200 may apply the second pulse sequence using a short TR and a short TE to the second slice. For example, the second pulse sequence may use a RR interval or a period equal to or less than the RR interval as one TR. Thus, the MRI apparatus 200 may not consider the second slice in the first pulse sequence. The second pulse sequence applied to the second slice by the MRI apparatus 200 will be described below with reference to FIG. 15.

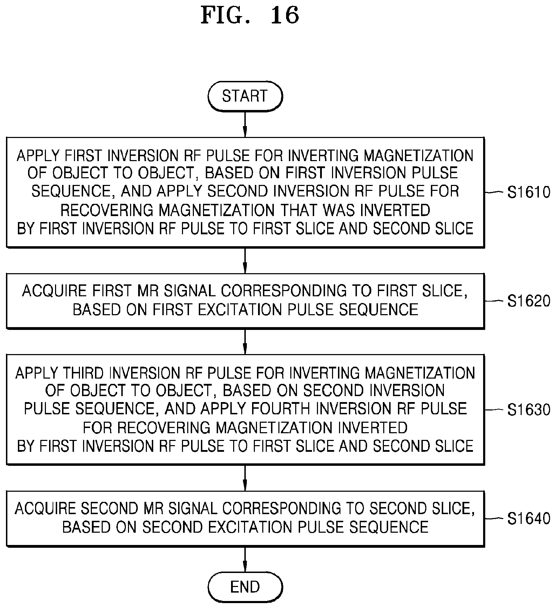

According to another exemplary embodiment, the MRI apparatus 200 may apply a second pulse sequence using a long TR and a short TE to the second slice. In this case, in order to minimize an influence of the first pulse sequence on the second slice during the first pulse sequence, the MRI apparatus 200 may further add, to the first pulse sequence, an RF pulse with respect to the second slice. A method, performed by the MRI apparatus 200, of applying the second pulse sequence using a long TR to the second slice will be described below with reference to FIG. 16.

When the second pulse sequence is applied to the second slice, the MRI apparatus 200 may acquire a second MR signal corresponding to the second slice. In this regard, the second MR signal may have a large signal difference between tissues, due to the use of a short TE.

In operation S1330, the MRI apparatus 200 may reconstruct a first MR image from the first MR signal, and may reconstruct a second MR image from the second MR signal. The MRI apparatus 200 may reconstruct the first MR image and the second MR image having different contrasts from the first MR signal and the second MR signal, respectively.

The MRI apparatus 200 may reconstruct, from the first MR signal, a T2-weighted image in which contrast between blood flow and a tissue is high. Also, the MRI apparatus 200 may reconstruct, from the second MR signal, a T1-weighted image in which contrast between tissues is high.

In exemplary embodiments, when the MRI apparatus 200 applies the second pulse sequence using a long TR and a short TE to the second slice, the MRI apparatus 200 may reconstruct a PD-weighted image from the second MR signal.

The MRI apparatus 200 may use various reconstruction techniques in order to reconstruct an MR image from an MR signal. For example, the MRI apparatus 200 may reconstruct an MR image by using the SENSE technique, the GRAPPA technique, or the like.

FIG. 14 is a flowchart of a method, performed by the MRI apparatus 200, of acquiring a first MR signal based on a first pulse sequence, according to an exemplary embodiment.

Referring to FIG. 14, in operation S1410, the MRI apparatus 200 may apply a first inversion RF pulse for inverting magnetization of an object to the object, based on a first inversion pulse sequence, and may apply a second inversion RF pulse for recovering the magnetization inverted by the first inversion RF pulse to a first slice. In this regard, an inversion RF pulse may be a 180° RF pulse.

According to the present exemplary embodiment, the MRI apparatus 200 may not generate a space encoding gradient magnetic field corresponding to the first inversion RF pulse, thereby allowing the first inversion RF pulse to influence all regions of the object.

When the second inversion RF pulse is applied to the object, the MRI apparatus 200 may generate a space encoding gradient magnetic field corresponding to the first slice. In this manner, the MRI apparatus 200 applies the second inversion RF pulse to the first slice, thereby recovering, in the first slice, the magnetization inverted by the first inversion RF pulse to an original state.

In operation S1420, the MRI apparatus 200 may acquire the first MR signal corresponding to the first slice, based on a first excitation pulse sequence. The MRI apparatus 200 may apply the space encoding gradient magnetic field corresponding to the first slice to the object, based on the first excitation pulse sequence, thereby acquiring the first MR signal corresponding to the first slice.

In exemplary embodiments, the MRI apparatus 200 may apply, to the first slice, a third inversion RF pulse for suppressing a fat signal, based on the first excitation pulse sequence, and may apply at least one excitation RF pulse to the first slice. In this case, the MRI apparatus 200 may apply the third inversion RF pulse to the first slice and then may acquire, or readout, the first MR signal by using TI of fat.

However, the third inversion RF pulse of the first excitation pulse sequence may be omitted. In this case, the MRI apparatus 200 may acquire the first MR signal by using TI of blood.

FIG. 15 is a flowchart of a method, performed by the MRI apparatus 200, of applying a second pulse sequence to a second slice, according to an exemplary embodiment.

Referring to FIG. 15, in operation S1510, the MRI apparatus 200 may apply a first inversion RF pulse for inverting magnetization of an object to the object, based on a second inversion pulse sequence, and may apply a second inversion RF pulse for recovering the magnetization that was inverted by the first inversion RF pulse to a first slice and a second slice.

According to the present exemplary embodiment, the MRI apparatus 200 may not generate a space encoding gradient magnetic field corresponding to the first inversion RF pulse, thereby allowing the first inversion RF pulse to influence all regions of the object.

When the second inversion RF pulse is applied to the object, the MRI apparatus 200 may generate a space encoding gradient magnetic field corresponding to the second slice. In this manner, the MRI apparatus 200 applies the second inversion RF pulse to the second slice, thereby recovering magnetization that was inverted by the first inversion RF pulse to an original state in the second slice.

In addition, when the second inversion RF pulse is applied to the object, the MRI apparatus 200 may further generate a space encoding gradient magnetic field corresponding to the first slice. This further generation may occur because a first pulse sequence uses a long TR. Atomic nuclei that were excited by the first pulse sequence and are not recovered in the first slice may be affected by the first inversion RF pulse. Thus, the MRI apparatus 200 may apply the second inversion RF pulse to the first slice, thereby recovering magnetization of the first slice to an original state.

In operation S1520, the MRI apparatus 200 may apply at least one excitation RF pulse to the second slice, based on a second excitation pulse sequence. The MRI apparatus 200 applies a space encoding gradient magnetic field corresponding to the second slice to the object, based on the second excitation pulse sequence, thereby acquiring a second MR signal corresponding to the second slice.

The MRI apparatus 200 may acquire the second MR signal by using TI of blood.

FIG. 16 is a flowchart of a method, performed by the MRI apparatus 200, of applying a first pulse sequence and a second pulse sequence which use a long TR to an object, according to another exemplary embodiment.

Referring to FIG. 16, in operation S1610, the MRI apparatus 200 may apply a first inversion RF pulse for inverting magnetization of the object to the object, based on a first inversion pulse sequence, and may apply a second inversion RF pulse for recovering the magnetization that was inverted by the first inversion RF pulse to a first slice and a second slice.

When the second pulse sequence using a long TR is applied to the second slice, the MRI apparatus 200 may apply the second inversion RF pulse to the second slice in order to minimize an influence of the first inversion RF pulse on the second slice in the first pulse sequence.

In operation S1620, the MRI apparatus 200 may acquire a first MR signal corresponding to the first slice, based on a first excitation pulse sequence. In this regard, a first MR image reconstructed based on the first MR signal may be a T2-weighted image.

In operation S1630, the MRI apparatus 200 may apply a third inversion RF pulse for inverting magnetization of the object to the object, based on a second inversion pulse sequence, and may apply a fourth inversion RF pulse for recovering the magnetization inverted by the first inversion RF pulse to the first slice and the second slice.

Because the first pulse sequence uses a long TR, the MRI apparatus 200 may apply the fourth inversion RF pulse to the first slice in order to minimize an influence of the third inversion RF pulse on the first slice in the second pulse sequence.

In operation S1640, the MRI apparatus 200 may acquire a second MR signal corresponding to the second slice, based on a second excitation pulse sequence. In this regard, a second MR image reconstructed based on the second MR signal may be a PD-weighted image.

Exemplary embodiments may be implemented through non-transitory computer-readable recording media having recorded thereon computer-executable instructions and data. The instructions may be stored in the form of program codes, and when executed by a processor, generate a predetermined program module to perform a specific operation. Furthermore, when executed by the processor, the instructions may perform specific operations according to exemplary embodiments.

While one or more exemplary embodiments have been described with reference to the figures, it will be understood by one of ordinary skill in the art that various changes in form and details may be made therein without departing from the scope of the present disclosure as defined by the following claims. Accordingly, the above exemplary embodiments and all aspects thereof are examples only and are not limiting.

**Claims**

1. A magnetic resonance imaging (MRI) apparatus comprising a processor and a memory connected to the processor, wherein the processor is configured to:

   control a radio frequency (RF) coil to apply a first pulse sequence to a first slice from among a plurality of slices of an object during a first RR interval of a heart, and acquire a first magnetic resonance (MR) signal corresponding to the first pulse sequence from the RF coil;
   control the RF coil to apply a second pulse sequence to a second slice from among the plurality of slices during a second RR interval of the heart, and acquire a second MR signal corresponding to the second pulse sequence from the RF coil; and
   reconstruct a first MR image from the first MR signal, and reconstruct a second MR image from the second MR signal.

2. The MRI apparatus of claim 1, wherein the first pulse sequence uses a long echo time (TE), and the second pulse sequence uses a short TE.

3. The MRI apparatus of claim 1 or 2, wherein the first pulse sequence comprises a first inversion pulse sequence for suppressing a blood signal in the first slice, and a first excitation pulse sequence for acquiring the first MR signal, and wherein the second pulse sequence comprises a second inversion pulse sequence for suppressing the blood signal in the first slice, and a second excitation pulse sequence for acquiring the second MR signal.

4. The MRI apparatus of claim 3, wherein, in the first inversion pulse sequence, the processor is further configured to control the RF coil to apply a first inversion RF pulse to the object for inverting a magnetization of the object, and to apply a second inversion RF pulse to the first slice for recovering the magnetization inverted by the first inversion RF pulse.

5. The MRI apparatus of claim 3, wherein, in the second inversion pulse sequence, the processor is further configured to control the RF coil to apply a first inversion RF pulse to the object to invert magnetization of the object, and to apply a second inversion RF pulse to the first slice and the second slice to recover the magnetization inverted by the first inversion RF pulse.

6. The MRI apparatus of any of the claims 3-5, wherein the first MR image is a T2-weighted image and the second MR image is a T1-weighted image.

7. The MRI apparatus of claim 3, wherein, in the first excitation pulse sequence, the processor is further configured to control the RF coil to apply to the first slice a third inversion RF pulse for suppressing a fat signal, and to apply to the first slice at least one RF excitation pulse for acquiring the first MR signal.

8. The MRI apparatus of claim 3 or 7, wherein, in the first inversion pulse sequence, when the second pulse sequence uses a long repetition time (TR), the processor is further configured control the RF coil to apply to the object a first inversion RF pulse for inverting magnetization of the object, and to apply to the first slice and the second slice a second inversion RF pulse for recovering the magnetization inverted by the first inversion RF pulse.

9. The MRI apparatus of claim 3, 7, or 8, wherein the first MR image is a T2-weighted image and the second MR image is a proton density (PD)-weighted image.

10. The MRI apparatus of any of the previous claims, wherein a position of the second slice is discontinuous with respect to a position of the first slice.

11. A method of operating a magnetic resonance imaging (MRI) apparatus, the method comprising:

applying, using a radio-frequency (RF) coil, a first pulse sequence to a first slice from among a plurality of slices of an object during a first RR interval of a heart;
acquiring, using the RF coil, a first magnetic resonance (MR) signal corresponding to the first pulse sequence;
applying, using the RF coil, a second pulse sequence to a second slice from among the plurality of slices during a second RR interval of the heart;
acquiring, using the RF coil, a second MR signal corresponding to the first pulse sequence;
reconstructing a first MR image from the first MR signal; and
reconstructing a second MR image from the second MR signal.

12. The method of claim 11, wherein the first pulse sequence uses a long echo time (TE), and the second pulse sequence uses a short TE.

13. The method of claim 11 or 12, wherein the first pulse sequence comprises a first inversion pulse sequence for suppressing a blood signal in the first slice, and a first excitation pulse sequence for acquiring the first MR signal, and wherein the second pulse sequence comprises a second inversion pulse sequence for suppressing the blood signal in the first slice, and a second excitation pulse sequence for acquiring the second MR signal.

14. The method of claim 13, wherein the acquiring of the first MR signal comprises:

in the first inversion pulse sequence, applying to the object a first inversion radio frequency (RF) pulse for inverting magnetization of the object, and applying to the first slice a second inversion RF pulse for recovering the magnetization inverted by the first inversion RF pulse; and
acquiring the first MR signal corresponding to the first slice, based on the first excitation pulse sequence.

15. A computer-readable recording medium having recorded thereon a program which, when executed, causes a processor to perform the method of any of the claims 11-14.

FIG. 1

EP 3 309 575 A1

# FIG. 2

# FIG. 3

NUMERAL OF SECOND SLICE = NUMERAL OF FIRST SLICE + TOTAL NUMBER OF SLICES /2

# FIG. 4

FIRST R WAVE     SECOND R WAVE     THIRD R WAVE     FOURTH R WAVE     400

FIRST RR INTERVAL (411)     SECOND RR INTERVAL (412)     THIRD RR INTERVAL (413)

# FIG. 5

FIRST RR INTERVAL (411)  SECOND RR INTERVAL (412)

501  502  first MR signal acquisition  503  504  second MR signal acquisition

TX

FIRST INVERSION PULSE SEQUENCE (511)  FIRST EXCITATION PULSE SEQUENCE (512)  SECOND INVERSION PULSE SEQUENCE (521)  SECOND EXCITATION PULSE SEQUENCE (522)

FIRST PULSE SEQUENCE (510)  SECOND PULSE SEQUENCE (520)

EP 3 309 575 A1

# FIG. 6

FIRST RR INTERVAL (411)

FIRST PULSE SEQUENCE (610)

FIRST INVERSION
PULSE SEQUENCE (611)

FIRST EXCITATION
PULSE SEQUENCE (612)

601  602      603

FIRST MR SIGNAL
ACQUISITION

TX

631        633    632        635    634        635    636

637

FIG. 7

# FIG. 8

FIRST PULSE SEQUENCE (801)

SECOND PULSE SEQUENCE (802)

811  812

821  822

FIRST MR SIGNAL ACQUISITION

SECOND MR SIGNAL ACQUISITION

RF

—— FIRST SLICE (810)
-·-· SECOND SLICE (820)

TISSUE

830-1

831  832

834  835

M0

time

BLOOD

830-2

831

834

M0

time

833

836

EP 3 309 575 A1

# FIG. 9

FIRST PULSE SEQUENCE (901)

SECOND PULSE SEQUENCE (902)

911 912 913

FIRST MR SIGNAL ACQUISITION

SECOND MR SIGNAL ACQUISITION

RF

— FIRST SLICE (910)
-·-· SECOND SLICE (920)

TISSUE

930-1

931

$M0$

932

time

BLOOD

930-2

$M0$

time

EP 3 309 575 A1

FIG. 10

First MR Signal Acquisition

First Excitation Pulse Sequence (1012)

First Inversion Pulse Sequence (1011)

First Pulse Sequence (1010)

Third RR Interval (1003)

Second MR Signal Acquisition

Second Excitation Pulse Sequence (1022)

Second Inversion Pulse Sequence (1021)

Second Pulse Sequence (1020)

Second RR Interval (1002)

First MR Signal Acquisition

First Excitation Pulse Sequence (1012)

First Inversion Pulse Sequence (1011)

First Pulse Sequence (1010)

First RR Interval (1001)

TX

TR    TR

# FIG. 11

SECOND RR INTERVAL (1002)    THIRD RR INTERVAL (1003)

FIRST PULSE SEQUENCE (1110)

FIRST INVERSION PULSE
SEQUENCE (1111)

FIRST EXCITATION
PULSE SEQUENCE (1112)

1101 1102

FIRST MR SIGNAL
ACQUISITION

TX

1131    1134  1133  1132    1136    1135

# FIG. 12

# FIG. 13

START

APPLY FIRST PULSE SEQUENCE TO FIRST SLICE FROM AMONG PLURALITY OF SLICES DURING FIRST RR INTERVAL OF HEART, THEREBY ACQUIRING FIRST MR SIGNAL — S1310

APPLY SECOND PULSE SEQUENCE TO SECOND SLICE FROM AMONG PLURALITY OF SLICES DURING SECOND RR INTERVAL OF HEART, THEREBY ACQUIRING SECOND MR SIGNAL — S1320

RECONSTRUCT FIRST MAGNETIC RESONANCE IMAGE FROM FIRST MR SIGNAL, AND RECONSTRUCT SECOND MAGNETIC RESONANCE IMAGE FROM SECOND MR SIGNAL — S1330

END

# FIG. 14

START

APPLY FIRST INVERSION RF PULSE FOR INVERTING MAGNETIZATION OF OBJECT TO OBJECT, BASED ON FIRST INVERSION PULSE SEQUENCE, AND APPLY SECOND INVERSION RF PULSE FOR RECOVERING MAGNETIZATION INVERTED BY FIRST INVERSION RF PULSE TO FIRST SLICE — S1410

ACQUIRE FIRST MR SIGNAL CORRESPONDING TO FIRST SLICE, BASED ON FIRST EXCITATION PULSE SEQUENCE — S1420

END

# FIG. 15

```
           ( START )
               │
               ▼
┌─────────────────────────────────────────┐
│ APPLY FIRST INVERSION RF PULSE FOR       │
│ INVERTING MAGNETIZATION                  │
│ OF OBJECT TO OBJECT, BASED ON SECOND     │
│ INVERSION                                │
│ PULSE SEQUENCE, AND APPLY SECOND         │── S1510
│ INVERSION RF PULSE                       │
│ FOR RECOVERING MAGNETIZATION THAT        │
│ WAS INVERTED                             │
│ BY FIRST INVERSION RF PULSE TO FIRST     │
│ SLICE AND SECOND SLICE                   │
└─────────────────────────────────────────┘
               │
               ▼
┌─────────────────────────────────────────┐
│ APPLY AT LEAST ONE EXCITATION RF PULSE   │
│ TO SECOND SLICE,                         │── S1520
│ BASED ON SECOND EXCITATION PULSE         │
│ SEQUENCE                                 │
└─────────────────────────────────────────┘
               │
               ▼
           (  END  )
```

# FIG. 16

START

APPLY FIRST INVERSION RF PULSE FOR INVERTING MAGNETIZATION OF OBJECT TO OBJECT, BASED ON FIRST INVERSION PULSE SEQUENCE, AND APPLY SECOND INVERSION RF PULSE FOR RECOVERING MAGNETIZATION THAT WAS INVERTED BY FIRST INVERSION RF PULSE TO FIRST SLICE AND SECOND SLICE — S1610

ACQUIRE FIRST MR SIGNAL CORRESPONDING TO FIRST SLICE, BASED ON FIRST EXCITATION PULSE SEQUENCE — S1620

APPLY THIRD INVERSION RF PULSE FOR INVERTING MAGNETIZATION OF OBJECT TO OBJECT, BASED ON SECOND INVERSION PULSE SEQUENCE, AND APPLY FOURTH INVERSION RF PULSE FOR RECOVERING MAGNETIZATION INVERTED BY FIRST INVERSION RF PULSE TO FIRST SLICE AND SECOND SLICE — S1630

ACQUIRE SECOND MR SIGNAL CORRESPONDING TO SECOND SLICE, BASED ON SECOND EXCITATION PULSE SEQUENCE — S1640

END

Europäisches
Patentamt

European
Patent Office

Office européen
des brevets

# EUROPEAN SEARCH REPORT

Application Number

EP 17 19 5936

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SEONG-EUN KIM ET AL: "Triple contrast technique for black blood imaging with double inversion preparation", MAGNETIC RESONANCE IN MEDICINE., vol. 52, no. 6, 23 November 2004 (2004-11-23), pages 1379-1387, XP055457525, US ISSN: 0740-3194, DOI: 10.1002/mrm.20296 * figure 1 * * table 1 * * section "MATERIALS AND METHODS" * | 1-15 | INV. G01R33/561 G01R33/56 G01R33/567 |
| X | DENNIS L. PARKER ET AL: "Improved efficiency in double-inversion fast spin-echo imaging", MAGNETIC RESONANCE IN MEDICINE., vol. 47, no. 5, 22 April 2002 (2002-04-22), pages 1017-1021, XP055457631, US ISSN: 0740-3194, DOI: 10.1002/mrm.10152 * figure 1 * * section "METHODS" * * section "CONCLUSIONS" * | 1-5,7-15 | |
| X | KIM ET AL.: "Implementation of Time Efficient Double Inversion FSE technique on a 3.0T system", PROCEEDINGS OF THE INTERNATIONAL SOCIETY FOR MAGNETIC RESONANCE IN MEDICINE, ISMRM, 13TH SCIENTIFIC MEETING AND EXHIBITION, MIAMI BEACH, FLORIDA, USA, 7-13 MAY 2005, 23 April 2005 (2005-04-23), XP040595050, * the whole document * | 1,3-5, 7-11, 13-15 | |

**TECHNICAL FIELDS SEARCHED (IPC)**

G01R
A61B

The present search report has been drawn up for all claims

| Place of search | Date of completion of the search | Examiner |
|---|---|---|
| Munich | 9 March 2018 | Durst, Markus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)